# EUROPEAN PATENT APPLICATION

(11) **EP 2 962 732 A1**
(43) Date of publication of application: **06.01.2016**
(21) Application number: 13876552.4
(22) Date of filing: 28.02.2013
(51) Int. Cl.: A61Q 7/00

(54) **PRODUCT FOR TOPICAL USE ON THE SCALP AND METHOD FOR PRODUCING SAME**

(71) Applicant: Jimeno Martínez, Leonardo, 24007 León (ES)
(72) Inventor: Jimeno Martínez, Leonardo, 24007 León (ES)
(74) Representative: Gallego Jiménez, José Fernando
(86) International application number: PCT/ES2013/070127
(87) International publication number: WO 2014/131919

(57) **Abstract**

The invention relates to a product for topical use on the scalp and to a method for producing same, consisting of a mixture of natural substances, comprising: palmitic acid, stearic acid, oleic acid, linoleic acid, ricinoleic acid, raw egg yolk, sugarcane alcohol and distilled water for the production thereof. The mixture of substances is beaten at a temperature of between 12 and 18°C, left to rest for 30 minutes, beaten again for 5 more minutes, cooled to 8°C, strained, left to macerate for 30 days in a crystal container, introduced into white glass flasks and left to rest at room temperature for 5 days.

## Description

### OBJECT OF THE INVENTION

As stated in this specification, the present invention relates to a product for topical use on the scalp and a method for producing the same.

More particularly, the object of the invention is directed to a composite product composed entirely of natural substances whose purpose is its application by massage, and according to a particular mode of application to the scalp of users, it being remarkably effective in preventing baldness, seborrhoea, dermatitis, dandruff or other scalp conditions, as well as to achieve or maintain healthy, strong and high-volume hair.

### SCOPE OF THE INVENTION

The scope of the present invention is part of the industrial sector engaged in the manufacture of topical use hair products.

### BACKGROUND OF THE INVENTION

As reference to the current state of the art, it should be noted that while there are many products applicable to solve various hair problems in the market, many of them specific for baldness problems, most of which are composition-based products comprising chemical or synthetic substances, the applicant is unaware of the existence of other similarly applied products or inventions which have technical and constitutive features similar to those of the product and production method presented herein, as claimed.

### EXPLANATION OF THE INVENTION

In summary, the invention suggests a product for topical use on the scalp whose main aim is to provide a solution to the hair loss problem suffered by many people, particularly, although not exclusively, men, since it provides an integral treatment avoiding, on the one hand, hair loss, and on the other hand, acting appropriately to achieve hair regeneration.

In summary, the product for topical use on the scalp stated by the invention is composed of a mixture of several substances of entirely natural origin, which comprises: palmitic acid, stearic acid, linoleic acid, oleic acid, sugarcane alcohol, ricinoleic acid, raw egg and distilled water.

In a preferred embodiment of the invention, said substances are mixed in the following ratios:
- Palmitic acid 0.35 %
- Stearic acid 0.38 %
- Linoleic acid 1.05 %
- Oleic acid 1.38 %
- Sugarcane alcohol 17.25 %
- Ricinoleic acid 23.57 %
- Egg yolk 27.27 %
- Distilled water 28.75 %

In turn, the method for manufacturing the product comprises the following steps:
Firstly, said substance mixture is beaten for at least 10 to 15 minutes at about 300 rpm in order for its homogenization process to be as uniform and complete as possible, all elements being at a temperature of at least between 12 and 18 °C.

Once the first step is over, the mixture is allowed to stand for about 30 minutes and then is continuously beaten again during 5 minutes.

Once the second beating step is completed, the product is introduced into a cooling apparatus to cool down to 8 °C.

Once the above step is completed, the mixture is passed through a fine strainer so that it becomes as liquid as possible, repeating this step if necessary.

Next, the obtained product is allowed to soak and rest for a period of 30 days, in a glass vial at all times.

Once the soak and rest period is over, the product is introduced into white glass flasks, in different doses as appropriate, and allowed to rest at room temperature during 5 days before being used and/or sold.

Regarding the mode of application of the product object of the invention, the steps that should be followed to obtain optimum results are the following:
It should be applied at night, preferably before going to bed, onto the scalp, mainly onto the areas most affected by hair loss by means of a soft massage with the fingertips embedded in the product, in a considerable amount, massaging then the rest of the scalp during 3-6 minutes and allowing the product to act overnight.

In order to avoid staining sheets and to increase the product's effectiveness, the head may be covered by a shower plastic cap that is to be kept during the night.

When waking up in the morning, the scalp should be washed with a neutral shampoo, preferably not damaging to the hair, glycerine or *lagarto* type soap may also be used and the hair is dried normally.

Once the treatment begins, its duration is recommended to be of 9 running days, that is, applying the product continuously during 9 nights. Once the 9 so-called "shock days" against baldness are over, the product should be continued to be used every two to three days during 15 more days.

Lastly, treatment should be interrupted, at least for a month, and after this period, the same process may be reinitiated. Once the first results are considered, that is, according to the result of the first shock plan, a longer or shorter rest period should be taken before new application processes are initiated, as necessary.

It should be noted that the invention product lacks side effects, since the new product is composed of non-damaging natural substances and lacks other synthetic or chemical products, no adverse side effects have been found in multiple tests carried out with the product.

Finally, it should be noted that the product described should be applied in case of baldness, seborrhoea, dermatitis, dandruff or other scalp diseases, which, in most cases, cause hair loss, therefore causing baldness.

The effect produced by the invention product is to eliminate the symptoms of said conditions, leaving the skin clean and healthy, the hair root thus receiving the vitamins and minerals required for normal activity. This product contains all essential nutrients for correct functioning of hair structure.

In view of the above, the product is indicated not only for those subjects suffering baldness but also for those seeking for healthy, strong and high-volume hair. In addition, the new hair that grows after applying the treatment is devoid of grey hair.

Likewise, the product acts from the first day of application, without exception. However, in order to achieve a good result, the product should be used at least once a week during nine months, since three types of hair begin to grow after this period: adult hair (that usually seen), intermediate hair (fine) and fluff (almost transparent hair, without melanin). In order for fluff to become adult hair, at least 1 month is required. In order for the treatment to be effective and good results be obtained, treatment exactly equal to that described above should be applied.

Having sufficiently described the nature of the present invention, as well as the manner of practicing it, it is not necessary to provide a more extensive explanation in order for its scope and advantages to be understood by any skilled person in the art, further noting that, within its essence, it may be practiced in other embodiments differing from those indicated as a way of example, which will also be covered by the protection sought, provided its main principle is not altered, changed or modified.

## Claims

1. Product for topical use on the scalp **characterized in that** it comprises a mixture of several natural substances, which comprises: palmitic acid, stearic acid, linoleic acid, oleic acid, sugarcane alcohol, ricinoleic acid, egg yolk and distilled water.

2. Product for topical use on the scalp, according to claim 1, **characterized in that** said substances are mixed in the following ratios:
- Palmitic acid 0.35 %
- Stearic acid 0.38 %
- Linoleic acid 1.05 %
- Oleic acid 1.38 %
- Sugarcane alcohol 17.25 %
- Ricinoleic acid 23.57 %
- Egg yolk 27.27 %
- Distilled water 28.75 %

3. Method for production of a product for topical use on the scalp as described in claim 1 or 2, **characterized in that** it comprises the following steps:
- The substance mixture is mixed during 10 to 15 minutes at about 300 rpm, all elements being at a temperature between at least 12 and 18 °C.
- It is allowed to rest for about 30 minutes.
- It is beaten again during 5 more minutes in a continuous manner.
- The mixed product is introduced in a cooling apparatus to cool down to 8 °C.
- It is passed through a fine strainer so that it becomes as liquid as possible, repeating this step if necessary.
- The obtained product is allowed to soak and rest for a period of 30 days, in a glass vial.
- The product is introduced into white glass flasks, at appropriate doses.
- It is allowed to rest at room temperature during 5 days, after which it is ready to be used and/or sold.
